# FASCICULE DE BREVET EUROPEEN

(11) **EP 1 086 951 B1**
(45) Date de publication et mention de la délivrance du brevet: **26.06.2002**
(21) Numéro de dépôt: 00402372.7
(22) Date de dépôt: 28.08.2000
(51) Int. Cl.: C07D 487/14, A61K 7/13, A61K 7/02

(54) **Nouveaux composés indoliques leur utilisation pour la teinture et le maquillage des matières kératiniques, compositions les contenant et procédés de teinture**
Indolverbindungen, ihre Verwendung zum Färben und Schminken von Keratinfasern, sie enthaltende Zusammensetzungen und Färbeverfanren
Indole compounds, their use for dyeing and make-up of keratinic materials , compositions containing them and dyeing processes

(30) Priorité: 22.09.1999 FR 9911833
(43) Date de publication de la demande: 28.03.2001
(73) Titulaire: L'OREAL, 75008 Paris (FR)
(72) Inventeur: Bonaventure, Nicole, 94300 Vincennes (FR); Gillard, Patrick, 93420 Villepinte (FR); Barre, Gilles, 93420 Villepinte (FR); Dubois, Michel, 77915 Pommeuse (FR)
(74) Mandataire: Miszputen, Laurent

(56) Documents cités:
- FR-A- 2 618 069
- FR-A- 2 677 544
- P. MANINI ET AL: "Acid-promoted competing pathways in the oxidative polymerization of 5,6-dihydroxyindoles and related compounds: straightforward cyclotrimerization routes to diindolocarbazole derivatives" JOURNAL OF ORGANIC CHEMISTRY, vol. 63, 1998, pages 7002-7008, XP002138534 EASTON US

## Description

FR 2618 069A, FR 2677 544A divulguent des dérivés de pigment(s) mélanique(s) obtenus par oxydation d'un composé indolique.

L'invention a pour objet de nouveaux composés indoliques, leur utilisation à titre de colorants directs dans des compositions destinées à la teinture des matières kératiniques et en particulier des compositions destinées à la teinture des fibres kératiniques humaines et notamment des cheveux, et dans des compositions cosmétiques destinées au maquillage de la peau, des ongles et des lèvres, les compositions de teinture ou de maquillage les contenant et le procédé de teinture directe correspondant.

Dans le domaine de la teinture capillaire, on recherche des colorants directs, c'est-à-dire des colorants qui, sans l'apport d'agent oxydant, sont capables de modifier par eux-mêmes la nuance naturelle des cheveux, de façon temporaire. Dans cette application, les colorants doivent satisfaire à un certain nombre de critères, et notamment engendrer des nuances riches et variées permettant d'obtenir une large palette de couleurs susceptible de satisfaire le formulateur ; dans cette optique, on recherche toujours de nouveaux composés permettant de teindre dans une gamme de couleur allant notamment de l'orangé au rouge. D'autre part, les teintures obtenues doivent être reproductibles, puissantes et résistantes au lavage, au frottement, à l'ondulation permanente, à la lumière et à la transpiration.

Or, la demanderesse vient maintenant de découvrir, de façon totalement inattendue et surprenante, de nouveaux composés indoliques de formule (I) ci-après définie, dans la gamme des nuances orangé à rouge, qui conviennent pour une utilisation comme colorant direct dans la teinture des matières kératiniques, et dans des compositions de maquillage de la peau, des ongles et des lèvres.
Ces nouveaux colorants permettent en outre d'obtenir des nuances orangé à rouge, puissantes, présentant d'excellentes propriétés de résistance aux différents traitements que peuvent subir les fibres kératiniques, et notamment les cheveux vis à vis de la lumière, des lavages, de l'ondulation permanente et de la transpiration.

Ces découvertes sont à la base de la présente invention.

L'invention a donc pour premier objet, de nouveaux composés de formule (I) suivante: 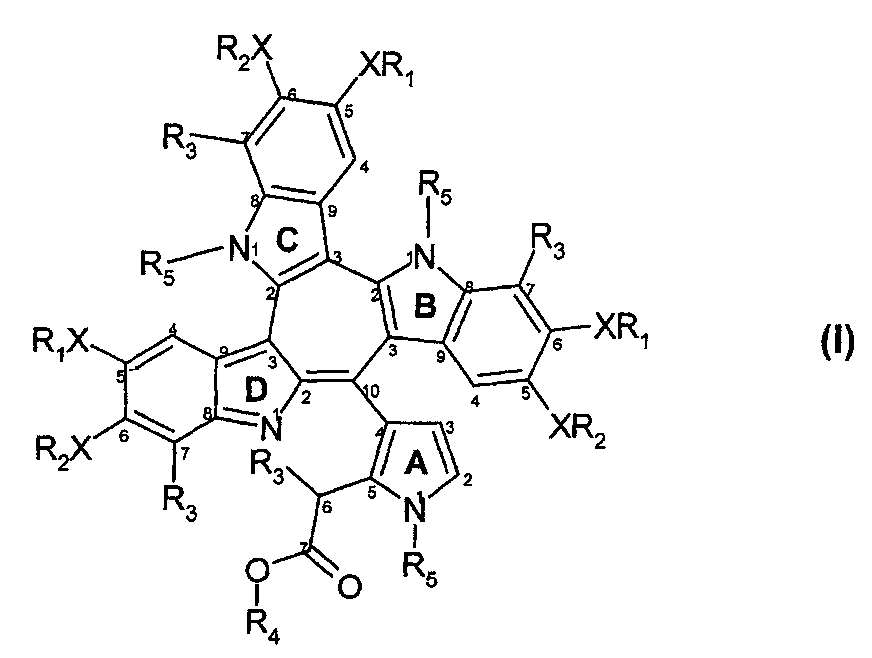 dans laquelle :
les radicaux X, identiques ou différents, désignent un atome d'oxygène ou d'azote,
R₁ et R₂, identiques ou différents, désignent un atome d'hydrogène, un radical alkyle en C₁-C₄, monohydroxyalkyle en C₁-C₄, polyhydroxyalkyle en C₁-C₄, acyl (alkyle en C₁-C₄ carbonyle), aminoalkyle en C₁-C₄, polyaminoalkyle en C₁-C₄, aminohydroxyalkyle en C₁-C₄,
R₃, R₄ et R₅, identiques ou différents, désignent un atome d'hydrogène ou une chaîne hydrocarbonée linéaire ou ramifiée, saturée ou insaturée en C₁-C₄.

Dans la formule (I) ci-dessus, le radical alkyle peut être linéaire ou ramifié et désigne notamment par exemple un radical méthyle, éthyle, n-propyle, isopropyle, n-butyle, isobutyle, ter.-butyle ; le radical monohydroxyalkyle en C₁-C₄ désigne plus particulièrement le radical -CH₂-CH₂OH et le radical polyhydroxyalkyle en C₁-C₄ désigne plus particulièrement le radical -CHOH-CH₂OH.

Parmi les composés indoliques de formule (I), on peut plus particulièrement citer le composé de formule (II) suivante :

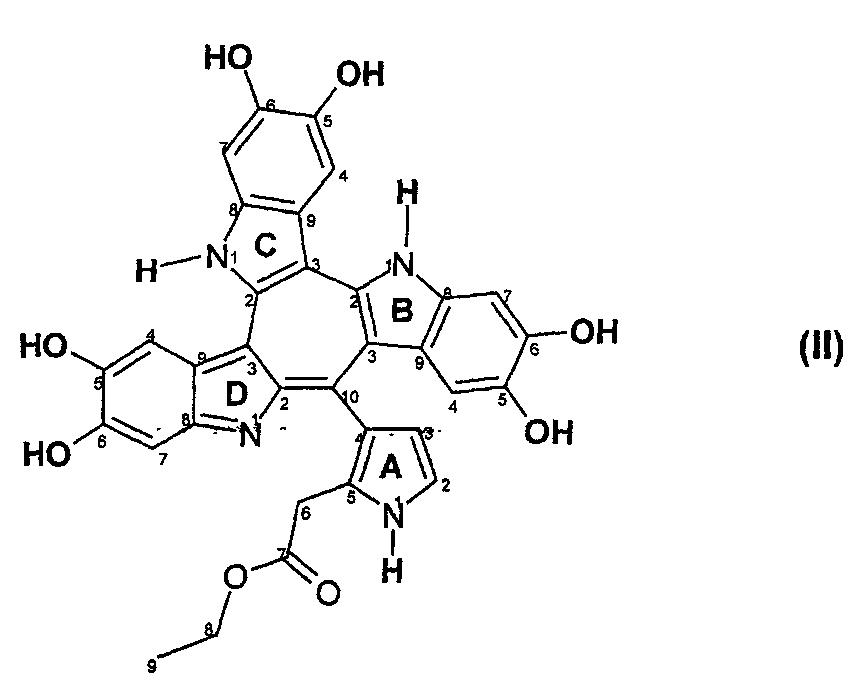

Les composés indoliques de formule (I) peuvent être obtenus par oxydation de l'indole correspondant en milieu solvant selon des procédés d'oxydation généralement bien connus de l'homme de l'art.
De cette façon, on oxyde en milieu alcoolique (méthanol, éthanol, n-propanol, isopropanol, n-butanol, terbutanol), et à température ambiante, en présence de métapériodate de sodium, un composé de formule (III) suivante : 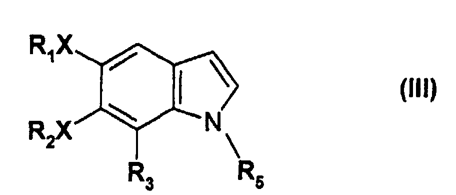 formule dans laquelle R₁, R₂, R₃ et R₅ et X ont les mêmes significations que dans la formule (I) décrite ci-dessus,
pour obtenir un composé de formule (I) selon l'invention.

On peut également remplacer le métapériodate de sodium par l'oxygène, le peroxyde d'hydrogène, le permanganate de potassium, l'oxyde de sélénium ou les peracides, en présence de catalyseurs métalliques tels que le manganèse, le fer ou le chrome.

L'invention a aussi pour objet un procédé de préparation d'un composé indolique de formule (I), par oxydation en milieu alcoolique d'un composé de formule (III) décrite ci-dessus, en présence de métapériodate de sodium, et à température ambiante.

L'invention a également pour objet l'utilisation des composés indoliques de formule (I) décrite ci-dessus, à titre de colorants directs dans des, ou pour la fabrication de, compositions de teinture ou de maquillage pour matières kératiniques, et notamment pour fibres kératiniques humaines telles que les cheveux.

L'invention a aussi pour objet une composition de teinture ou de maquillage des matières kératiniques, caractérisée par le fait qu'elle comprend, dans un milieu approprié pour la teinture ou le maquillage, une quantité efficace d'au moins un composé de formule (I) conforme à l'invention.

Au sens de la présente invention, on entend principalement par matières kératiniques, la peau du visage ou du corps, les lèvres, les ongles, les fibres kératiniques humaines telles que les cheveux humains, les poils, cils, sourcils, et également les fibres kératiniques telles que les fibres textiles naturelles dont notamment la laine.

L'invention a en particulier pour objet une composition de teinture directe des fibres kératiniques humaines telles que les cheveux, caractérisée par le fait qu'elle comprend, dans un milieu approprié pour la teinture, une quantité efficace d'au moins un composé de formule (I) conforme à l'invention.

Mais d'autres caractéristiques, aspects et avantages de l'invention apparaîtront encore plus clairement à la lecture de la description qui va suivre, ainsi que des divers exemples concrets, mais nullement limitatifs, destinés à l'illustrer.

Lorsque la composition est destinée à la teinture, le ou les composés de formule (I) conformes à l'invention représentent de préférence de 0,05 à 10 % en poids environ du poids total de la composition tinctoriale, et encore plus préférentiellement de 0,05 à 0,5 % en poids environ de ce poids.
Lorsque la composition est destinée au maquillage, le ou les composés de formule (I) conformes à l'invention représentent de préférence de 0,05 à 20 % en poids environ du poids total de la composition, et encore plus préférentiellement de 0,05 à 5 % en poids environ de ce poids.

Les composés de formule (I) conformes à l'invention peuvent également servir, dans les procédés bien connus de teinture d'oxydation des fibres kératiniques humaines, utilisant des colorants d'oxydation (précurseurs de colorants d'oxydation et éventuellement des coupleurs), à nuancer ou enrichir de reflets les teintures obtenues avec les colorants d'oxydation.

La composition tinctoriale selon l'invention peut encore contenir, pour élargir la palette de nuances et obtenir des teintes variées, outre les composés orangé à rouge de formule (I) selon l'invention, d'autres colorants directs classiquement utilisés, et notamment, des colorants nitrés benzéniques, comme les nitrophénylènediamines, les nitrodiphénylamines, les nitroanilines, les éthers de phénol nitrés ou les nitrophénols, des nitropyridines, des colorants anthraquinoniques, des colorants mono- ou di-azoïques, triarylméthaniques, aziniques, acridiniques et xanthéniques, ou encore des colorants métallifères.

La proportion de tous ces autres colorants directs d'addition peut varier entre 0,5 et 10% en poids environ par rapport au poids total de la composition tinctoriale.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.
Il peut aussi contenir des corps gras tels que des huiles et des cires.

Les solvants peuvent être présents dans des proportions de préférence comprises entre 1 et 70 % en poids environ par rapport au poids total de la composition tinctoriale, et encore plus préférentiellement entre 5 et 40 % en poids environ.

On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.
On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
On peut également utiliser des agents épaississants dans une proportion allant d'environ 0,2 à 5%.
La dite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Le pH de la composition tinctoriale conforme à l'invention destinée à la teinture des fibres kératiniques est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 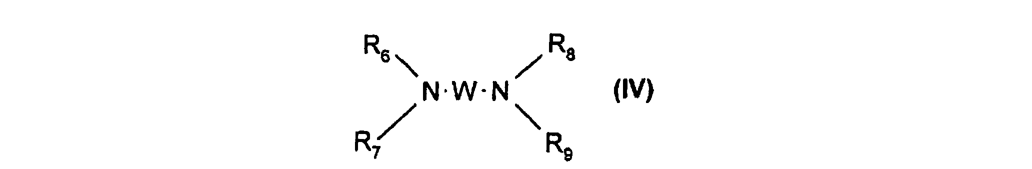 dans laquelle W est un reste propylène éventuellement substitué par un groupement hydroxyle ou un radical alkyle en C₁-C₆ ; R₆, R₇, R₈ et R₉, identiques ou différents, représentent un atome d'hydrogène, un radical alkyle en C₁-C₆ ou hydroxyalkyle en C₁-C₆.

La composition tinctoriale selon l'invention peut se présenter sous des formes diverses, telles que sous forme de liquides, de crèmes, de gels, de cataplasmes, ou sous toute autre forme appropriée pour réaliser une teinture des matières kératiniques, et notamment des cheveux humains. En particulier, elle peut être conditionnée sous pression en flacon aérosol en présence d'un agent propulseur et former une mousse.

Les compositions cosmétiques destinées au maquillage sont notamment des produits de maquillage pour le visage ou les lèvres tels que les fards à paupières ou à joue, les poudres et blushes, les fonds de teint, les bâtons ou laques à lèvres et les produits de maquillage du corps humain; elles sont aussi des produits de maquillage des cils, des sourcils et des ongles, tels que les mascaras, les crayons pour sourcils, les ligneurs encore appelés "eye-liner" et les vernis à ongles.

Un autre objet de l'invention porte sur un procédé de teinture des fibres kératiniques et en particulier des fibres kératiniques humaines telles que les cheveux, par coloration directe, consistant à laisser agir une composition renfermant au moins un composé de formule (I) selon l'invention sur les fibres kératiniques sèches ou humides.
On peut utiliser la composition selon l'invention en tant que composition non rincée, c'est-à-dire qu'après application de la composition sur les fibres, on sèche sans rinçage intermédiaire.
Dans les autres modes d'application, après application de la composition sur les fibres pendant un temps de pose variant entre 3 et 60 minutes environ, de préférence entre 5 et 45 minutes environ, on rince, éventuellement on lave, puis on rince à nouveau, et on sèche.

Des exemples concrets et non limitatifs illustrant l'invention vont maintenant être donnés.

### EXEMPLE 1 DE PREPARATION

Pour préparer le composé de formule (II) suivante : 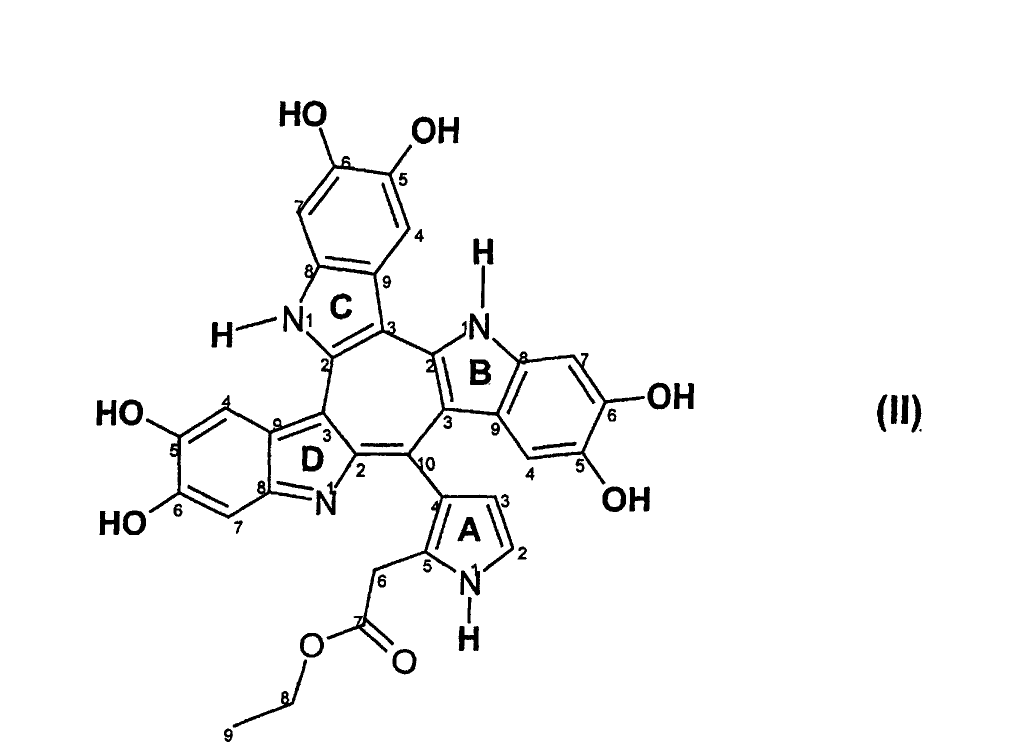 on a solubilisé 5 grammes (0,033 mole) de 5,6-dihydroxyindole dans 100 grammes d'éthanol. On a ajouté 7,18 grammes (0,033 mole) de métapériodate de sodium. On a laissé sous agitation et à température ambiante pendant 8 heures.
Après centrifugation et lavage, les phases organiques ont été réunies et évaporées à sec.
Le composé de formule (II) a été isolé par chromatographie HPLC préparative.
Après séchage à 40°C, on a isolé 50 mg de cristaux rouges. La structure du composé a été déterminée en RMN 1D ¹H, ¹³C, ¹⁵N et en RMN 2D ¹H-¹³C et ¹H-¹⁵N et en spectrométrie de masse.

**RMN:** Spectre RMN ¹³C (solvant : CD₃OD-*d*₄, référence : signal central du solvant à 49,00 ppm) : les déplacements chimiques ¹³C, les multiplicités (s : singulet, d : doublet, t : triplet, q : quadruplet) et les attributions sont donnés dans le tableau suivant :

| Déplacements chimiques ¹³C | | |
|---|---|---|
| δ en ppm | Multiplicité | Attribution |
| 13,83 | q | C-9 A |
| 32,97 | t | C-6 A |
| 62,02 | t | C-8 A |
| 97,89 | d | C-7 D |
| 99,02 | d | C-7 B |
| 99,47 | d | C-7 C |
| 107,65 | d | C-4 D |
| 108,57 | d | C-4 C |
| 109,51 | d | C-3 A |
| 111,23 | d | C-4 B |
| 115,09 | s | C-3 C |
| 116,18 | s | C-3 D |
| 116,37 | s | C-9 D |
| 117,09 | s | C-9 C |
| 118,10 | s | C-9 B |
| 119,36 | s | C-4 A |
| 121,12 | d | C-2 A |
| 122,59 | s | C-5 A |
| 128,73 | s | C-3 B |
| 133,33 | s | C-10 A |
| 137,56 | s | C-5 B |
| 137,67 | s | C-5 C |
| 137,73 | s | C-5 D |
| 139,76 | s | C-2 D |
| 140,80 | s | C-2 C |
| 143,06 | s | C-2 B |
| 143,12 | s | C-6 B |
| 144,42 | s | C-6 C |
| 144,98 | s | C-6 D |
| 149,74 | s | C-8 B |
| 149,79 | s | C-8 C |
| 151,66 | s | C-8 D |
| 171,97 | s | C-7 A |

### EXEMPLE 2 DE COMPOSITION TINCTORIALE

On a préparé la composition de teinture directe pour cheveux suivante:

| | |
|---|---|
| Composé de formule (II) 10⁻³ mole | 0,6 g |
| Alcool benzylique | 14 g |
| Ethanol | 46 g |
| Eau déminéralisée qsp | 100 g |

On a appliqué la composition ci-dessus sur des mèches de cheveux gris naturels ou permanentés à 90% de blancs à raison de 5 g de composition par gramme de cheveux et on a laissé poser pendant 30 minutes. Après rinçage à l'eau courante et séchage, les cheveux ont été teints dans une nuance rouge profond.

## Revendications

1. Composés indoliques de formule (I) suivante : 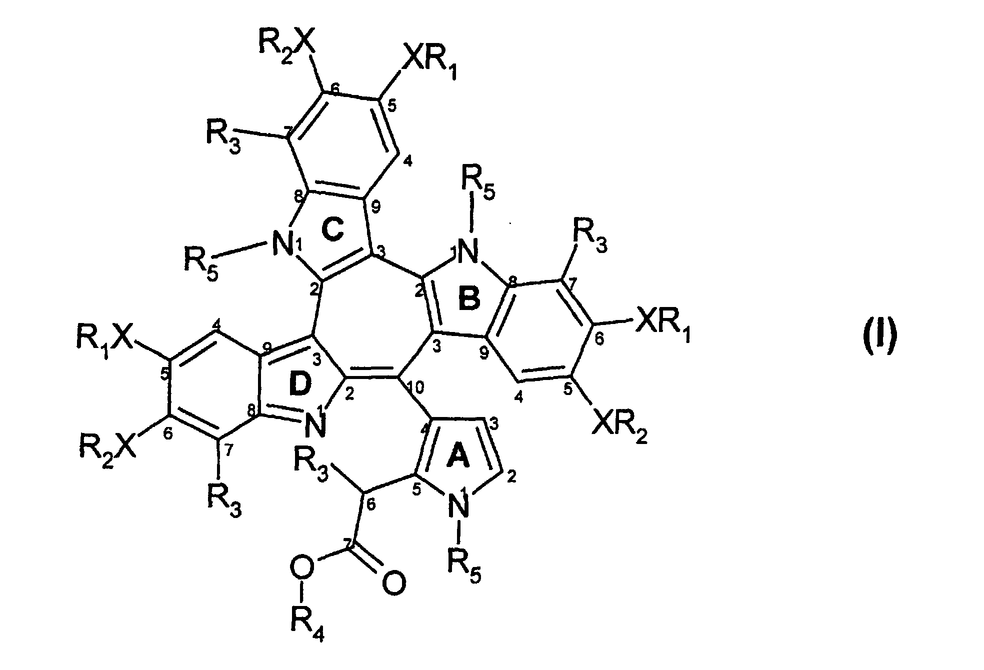 dans laquelle :
les radicaux X, identiques ou différents, désignent un atome d'oxygène ou d'azote,
R₁ et R₂, identiques ou différents, désignent un atome d'hydrogène, un radical alkyle en C₁-C₄, monohydroxyalkyle en C₁-C₄, polyhydroxyalkyle en C₁-C₄, acyl (alkyle en C₁-C₄ carbonyle), aminoalkyle en C₁-C₄, polyaminoalkyle en C₁-C₄, aminohydroxyalkyle en C₁-C₄,
R₃, R₄ et R₅, identiques ou différents, désignent un atome d'hydrogène ou une chaîne hydrocarbonée linéaire ou ramifiée, saturée ou insaturée en C₁-C₄.

2. Composé selon la revendication 1, de formule (II) suivante :

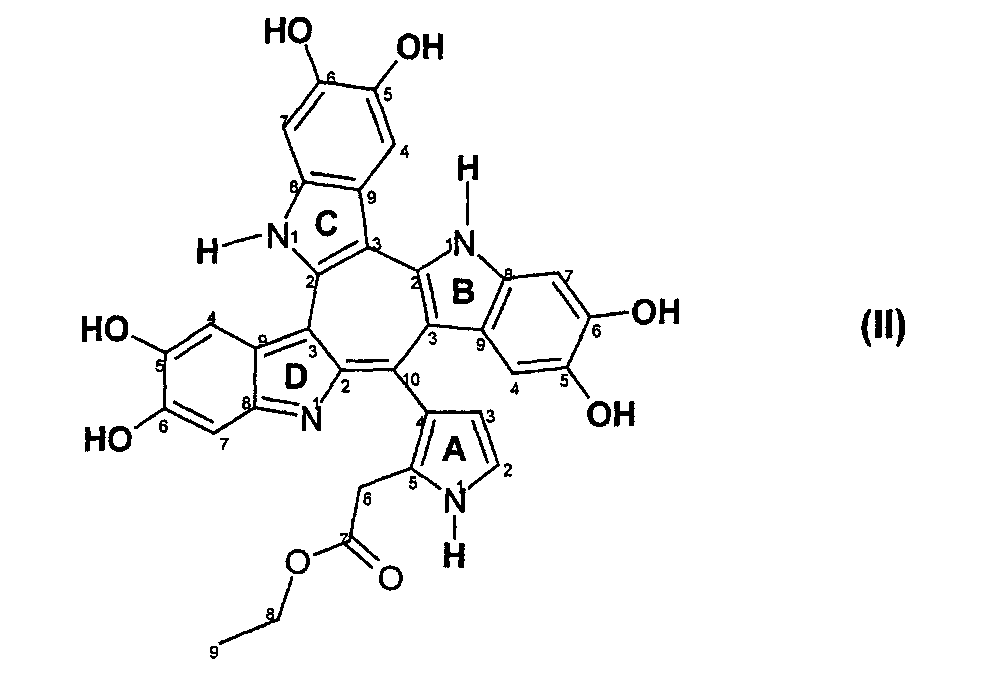

3. Utilisation à titre de colorants directs, ou pour la fabrication de, compositions tinctoriales ou de compositions de maquillage pour matières kératiniques, de composés de formule (I) selon la revendication 1.

4. Utilisation selon la revendication 3, **caractérisée par le fait que** les matières kératiniques désignent la peau humaine, les lèvres et les ongles.

5. Utilisation selon la revendication 3, **caractérisée par le fait que** les matières kératiniques désignent les fibres kératiniques humaines telles que les cheveux, les poils, les cils et les sourcils.

6. Composition de teinture ou de maquillage pour matières kératiniques, **caractérisée par le fait qu'**elle comprend, dans un milieu approprié pour la teinture ou le maquillage, une quantité efficace d'au moins un composé de formule (I) défini à la revendication 1.

7. Composition de teinture directe pour fibres kératiniques humaines et notamment les cheveux, **caractérisée par le fait qu'**elle comprend, dans un milieu approprié pour la teinture, une quantité efficace d'au moins un composé de formule (I) défini à la revendication 1.

8. Composition selon la revendication 7, **caractérisée par le fait qu'**elle a un pH compris entre 3 et 12.

9. Composition de teinture selon l'une quelconque des revendications 7 ou 8, **caractérisée par le fait que** les composés de formule (I) sont présents dans une concentration allant de 0,05 à 10 % en poids par rapport au poids total de la composition.

10. Composition selon la revendication 9, **caractérisée par le fait que** les composés de formule (I) sont présents dans une concentration allant de 0,05 à 0,5 % en poids par rapport au poids total de la composition.

11. Composition selon l'une quelconque des revendications 7 à 10, **caractérisée par le fait que** le milieu approprié pour la teinture est un milieu aqueux constitué par de l'eau et/ou des solvants organiques, les solvants étant présents dans des proportions comprises entre 1 et 70% en poids par rapport au poids total de la composition.

12. Composition de maquillage selon la revendication 6, **caractérisée par le fait que** les composés de formule (I) sont présents dans une concentration allant de 0,05 à 20 % en poids par rapport au poids total de la composition.

13. Composition selon la revendication 6 ou 12, **caractérisée par le fait qu'**elle se présente sous la forme d'un produit de maquillage de la peau humaine, des lèvres, des ongles, ou des cils et des sourcils.

14. Composition selon la revendication 13, **caractérisée par le fait qu'**elle se présente sous la forme d'un fond de teint, d'un fard à joue, ou à paupières, d'une poudre ou blush, d'un bâton ou laque à lèvres, d'un vernis à ongles, d'un mascara, d'un eye-liner.

15. Procédé de teinture des fibres kératiniques et en particulier des fibres kératiniques humaines et notamment les cheveux, par coloration directe, **caractérisé par le fait qu'**on applique une composition tinctoriale telle que définie à l'une quelconque des revendications 7 à 11, sur les fibres kératiniques sèches ou humides, et qu'on sèche ces fibres sans rinçage intermédiaire.

16. Procédé de teinture des fibres kératiniques et en particulier des fibres kératiniques humaines et notamment les cheveux, par coloration directe, **caractérisé par le fait qu'**on applique une composition tinctoriale telle que définie à l'une quelconque des revendications 7 à 11 sur les fibres kératiniques sèches ou humides, et qu'après avoir éventuellement laissé agir la composition sur les fibres pendant un temps de pose allant de 3 à 60 minutes, on rince les fibres, on les lave éventuellement, on les rince à nouveau puis on les sèche.

17. Procédé de préparation d'un composé indolique de formule (I) ou (II) selon les revendications 1 ou 2, **caractérisé par le fait qu'**on oxyde en milieu alcoolique un composé de formule (III) suivante : 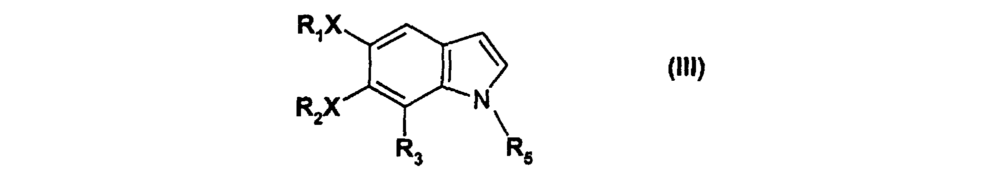 dans laquelle R₁, R₂, R₃ et R₅ et X ont les mêmes significations que dans la formule (I), en présence de métapériodate de sodium, et à température ambiante.

18. Procédé selon la revendication 17, **caractérisé par le fait que** le milieu alcoolique est l'alcool éthylique.

## Patentansprüche

1. Indolverbindungen der folgenden Formel (I): 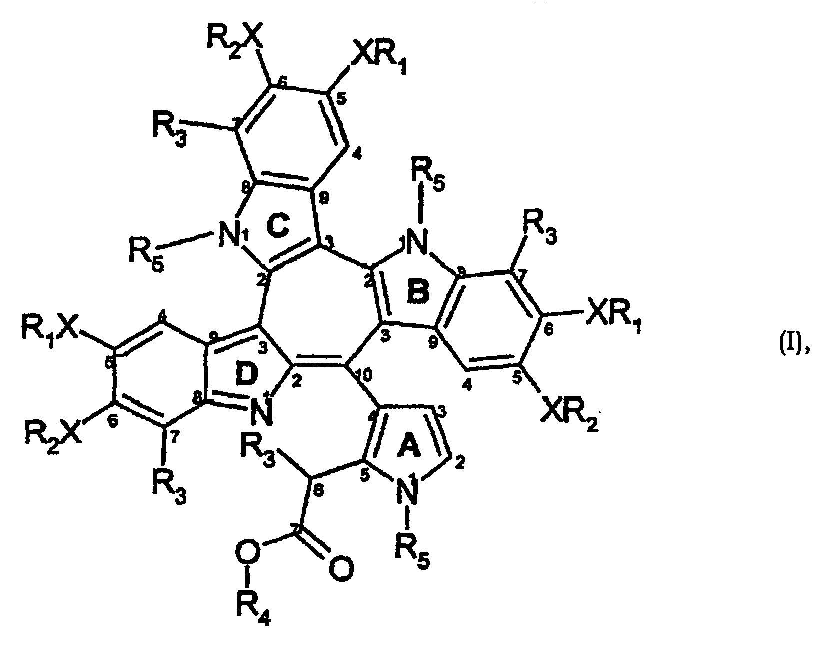 worin bedeuten:
- die Gruppen X, die identisch oder voneinander verschieden sind, Sauerstoff oder Stickstoff,
- die Gruppen R₁ und R₂, die identisch oder voneinander verschieden sind, Wasserstoff, C₁₋₄-Alkyl, C₁₋₄-Monohydroxyalkyl, C₁₋₄-Polyhydroxyalkyl, Acyl (C₁₋₄-Alkylcarbonyl), C₁₋₄-Aminoalkyl, C₁₋₄-polyaminoalkyl und C₁₋₄-Aminohydroxyalkyl; und
- die Gruppen R₃, R₄ und R₅, die identisch oder voneinander verschieden sind, Wasserstoff oder eine geradkettige oder verzweigte, gesättigte oder ungesättigte Kohlenwasserstoffkette mit 1 bis 4 Kohlenstoffatomen.

2. Verbindung nach Anspruch 1 der folgenden Formel (II):

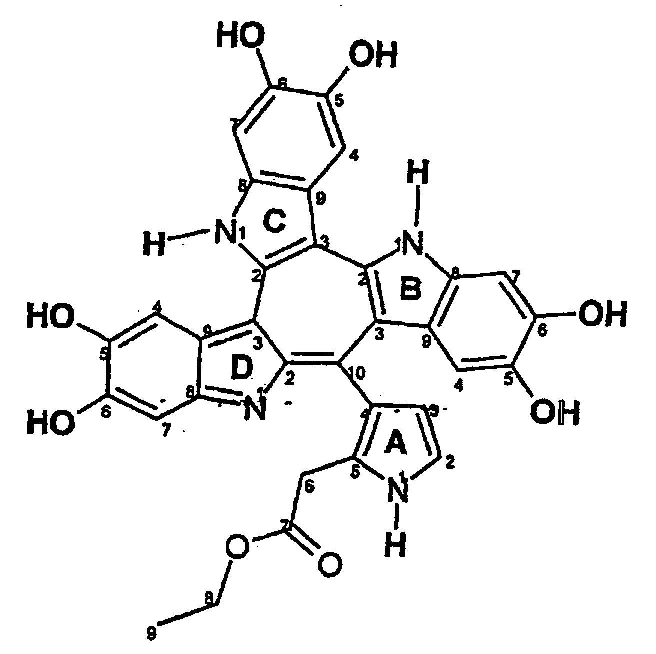

3. Verwendung von Verbindungen der Formel (I) nach Anspruch 1 als Direktfarbstoffe oder zur Herstellung von Zusammensetzungen zum Färben oder Zusammensetzungen zum Schminken von Keratinfasern.

4. Verwendung nach Anspruch 3, **dadurch gekennzeichnet, daß** die Keratinsubstanzen die menschliche Haut, die Lippen und die Nägel bedeuten.

5. Verwendung nach Anspruch 3, **dadurch gekennzeichnet, daß** die Keratinsubstanzen die menschlichen Keratinfasern, wie Haare, Körperhaar, Wimpern und Augenbrauen, bedeuten.

6. Zusammensetzung zum Färben oder zum Schminken von Keratinsubstanzen, **dadurch gekennzeichnet, daß** sie in einem zum Färben oder Schminken geeigneten Medium eine wirksame Menge mindestens einer Verbindung der Formel (I) nach Anspruch 1 enthält.

7. Zusammensetzung zum direkten Färben von menschlichen Keratinfasern und insbesondere Haaren, **dadurch gekennzeichnet, daß** sie in einem zum Färben geeigneten Medium eine wirksame Menge mindestens einer Verbindung der Formel (I) nach Anspruch 1 enthält.

8. Zusammensetzung nach Anspruch 7, **dadurch gekennzeichnet, daß** sie einen pH-Wert im Bereich von 3 bis 12 aufweist.

9. Zusammensetzung zum Färben nach einem der Ansprüche 7 oder 8, **dadurch gekennzeichnet, daß** die Verbindungen der Formel (I) in einer Konzentration im Bereich von 0,05 bis 10 Gew.-%, bezogen auf das Gesamtgewicht der Zusammensetzung, vorliegen.

10. Zusammensetzung nach Anspruch 9, **dadurch gekennzeichnet, daß** die Verbindungen der Formel (I) in einer Konzentration im Bereich von 0,05 bis 0,5 Gew.-%, bezogen auf das Gesamtgewicht der Zusammensetzung, vorliegen.

11. Zusammensetzung nach einem der Ansprüche 7 bis 10, **dadurch gekennzeichnet, daß** das zum Färben geeignete Medium ein wäßriges Medium ist, das aus Wasser und/oder organischen Lösungsmitteln besteht, wobei die Lösungsmittel in Mengenanteilen von 1 bis 70 Gew.-%, bezogen auf das Gesamtgewicht der Zusammensetzung, vorliegen.

12. Zusammensetzung zum Schminken nach Anspruch 6, **dadurch gekennzeichnet, daß** die Verbindungen der Formel (I) in einer Konzentration im Bereich von 0,05 bis 20 Gew.-%, bezogen auf das Gesamtgewicht der Zusammensetzung, vorliegen.

13. Zusammensetzung nach Anspruch 6 oder 12, **dadurch gekennzeichnet, daß** sie als Produkt zum Schminken der menschlichen Haut, der Lippen, der Nägel, der Wimpern oder der Augenbrauen vorliegt.

14. Zusammensetzung nach Anspruch 13, **dadurch gekennzeichnet, daß** sie als Make-up, Wangenrouge, Lidschatten, Puder, Rouge, Lippenstift, Lack für die Lippen, Nagellack, Mascara oder Eyeliner vorliegt.

15. Verfahren zum Färben von Keratinfasern, insbesondere menschlichen Keratinfasern und besonders dem Haar durch direkte Färbung, **dadurch gekennzeichnet, daß** eine Farbmittelzusammensetzung nach einem der Ansprüche 7 bis 11 auf die trockenen oder feuchten Keratinfasern aufgetragen und die Fasern ohne zwischenzeitlichen Spülschritt getrocknet werden.

16. Verfahren zum Färben von Keratinfasern und insbesondere menschlichen Keratinfasern und besonders Haaren durch direkte Färbung, **dadurch gekennzeichnet, daß** eine Farbmittelzusammensetzung nach einem der Ansprüche 7 bis 11 auf die trockenen oder feuchten Keratinfasern aufgetragen und die Fasern, nachdem die Zusammensetzung gegebenenfalls mit einer Einwirkzeit von 3 bis 60 min einwirken gelassen wurde, gespült, gegebenenfalls gewaschen, nochmals gespült und dann getrocknet werden.

17. Verfahren zur Herstellung einer Indolverbindung der Formel (I) oder (II) nach den Ansprüchen 1 oder 2, **dadurch gekennzeichnet, daß** in einem alkoholischen Medium eine Verbindung der folgenden Formel (III): 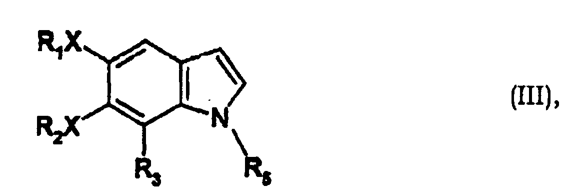 worin die Gruppen R₁, R₂, R₃ und R₅ und X die für Formel (I) angegebenen Bedeutungen aufweisen, in Gegenwart von Natriummetaperiodat bei Raumtemperatur oxidiert wird.

18. Verfahren nach Anspruch 17, **dadurch gekennzeichnet, daß** das alkoholische Medium Ethylalkohol ist.

## Claims

1. Indole compounds of formula (I) below: 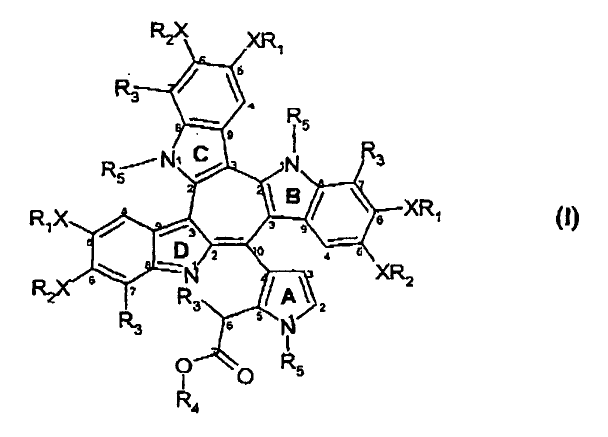 in which:
the radicals X, which may be identical or different, denote an oxygen or nitrogen atom,
R₁ and R₂, which may be identical or different, denote a hydrogen atom or a C₁-C₄ alkyl, C₁-C₄ monohydroxyalkyl, C₁-C₄ polyhydroxyalkyl, acyl {(C₁-C₄ alkyl)carbonyl}, C₁-C₄ aminoalkyl, C₁-C₄ polyaminoalkyl or C₁-C₄ aminohydroxyalkyl radical,
R₃, R₄ and R₅, which may be identical or different, denote a hydrogen atom or a linear or branched, saturated or unsaturated C₁-C₄ hydrocarbon-based chain

2. Compound according to Claim 1, of formula (II) below:

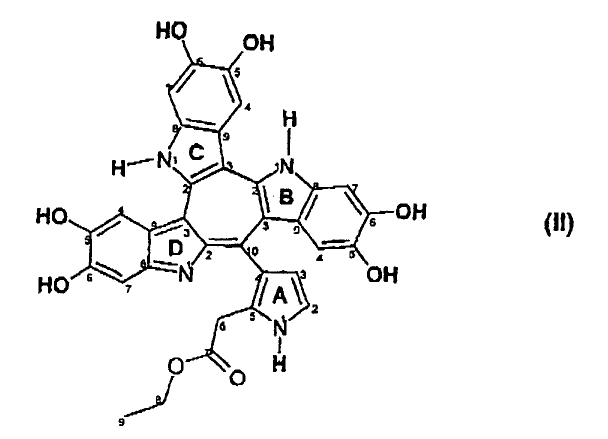

3. Use, as direct dyes or for the manufacture of dye compositions or make-up compositions for keratin materials, of compounds of formula (I) according to Claim 1.

4. Use according to Claim 3, **characterized in that** the keratin materials denote human skin, the lips and the nails.

5. Use according to Claim 3, **characterized in that** the keratin materials denote human keratin fibres such as the hair, body hairs, the eyelashes and the eyebrows.

6. Dye composition or make-up composition for keratin materials, **characterized in that** it comprises, in a medium which is suitable for dyeing or for make-up, an effective amount of at least one compound of formula (I) defined in Claim 1.

7. Direct dye composition for human keratin fibres and in particular the hair, **characterized in that** it comprises, in a medium which is suitable for dyeing, an effective amount of at least one compound of formula (I) defined in Claim 1.

8. Composition according to Claim 7, **characterized in that** it has a pH of between 3 and 12.

9. Dye composition according to either of Claims 7 and 8, **characterized in that** the compounds of formula (I) are present in a concentration ranging from 0.05% to 10% by weight relative to the total weight of the composition.

10. Composition according to Claim 9, **characterized in that** the compounds of formula (I) are present in a concentration ranging from 0.05% to 0.5% by weight relative to the total weight of the composition.

11. Composition according to any one of Claims 7 to 10, **characterized in that** the medium which is suitable for dyeing is an aqueous medium consisting of water and/or organic solvents, the solvents being present in proportions of between 1% and 70% by weight relative to the total weight of the composition.

12. Make-up composition according to Claim 6, **characterized in that** the compounds of formula (I) are present in a concentration ranging from 0.05% to 20% by weight relative to the total weight of the composition.

13. Composition according to Claim 6 or 12, **characterized in that** it is in the form of a make-up product for human skin, the lips, the nails, the eyelashes or the eyebrows.

14. Composition according to Claim 13, **characterized in that** it is in the form of a foundation, a face powder, an eyeshadow, a powder or blusher, a lipstick or lip gloss, a nail varnish, a mascara or an eyeliner.

15. Process for dyeing keratin fibres, and in particular human keratin fibres and especially the hair, by direct dyeing, **characterized in that** a dye composition as defined in any one of Claims 7 to 11 is applied to the wet or dry keratin fibres, and these fibres are dried without intermediate rinsing.

16. Process for dyeing keratin fibres, and in particular human keratin fibres and especially the hair, by direct dyeing, **characterized in that** a dye composition as defined in any one of Claims 7 to 11 is applied to the wet or dry keratin fibres, and, after the composition has optionally been left to act on the fibres for an exposure time ranging from 3 to 60 minutes, the fibres are rinsed, optionally washed, rinsed again and then dried.

17. Process for preparing an indole compound of formula (I) or (II) according to Claim 1 or 2, **characterized in that** a compound of formula (III) below: 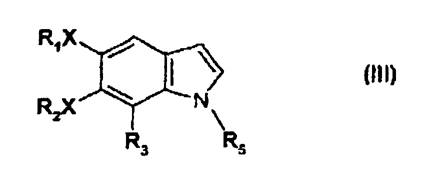 in which R₁, R₂, R₃ and R₅ and X have the same meanings as in formula (I), is oxidized in alcoholic medium, in the presence of sodium metaperiodate, and at room temperature.

18. Process according to Claim 17, **characterized in that** the alcoholic medium is ethyl alcohol.
